# EUROPEAN PATENT SPECIFICATION

(11) **EP 0 728 310 B1**
(45) Date of publication and mention of the grant of the patent: **04.08.1999**
(21) Application number: 95900689.1
(22) Date of filing: 08.11.1994
(51) Int. Cl.: G01N 33/74

(54) **ASSAY METHODS AND DEVICES THEREFOR**
NACHWEISVERFAHREN UND VORRICHTUNG DAFÜR
PROCEDES DE DOSAGE ET DISPOSITIFS POUR LEUR MISE EN OEUVRE

(30) Priority: 12.11.1993 EP 93309056
(43) Date of publication of application: 28.08.1996
(73) Proprietor: UNIPATH LIMITED, Basingstoke, Hampshire RG24 OPW (GB)
(72) Inventor: COLLINS, William, Patrick, Hertfordshire AL5 2BL (GB)
(74) Representative: Butler, David John
(86) International application number: EP9403702
(87) International publication number: WO9513543

(56) References cited:
- EP-A- 0 044 140
- EP-A- 0 086 095
- WO-A-87/02774
- WO-A-94/04926
- DATABASE WPI Week 8847, Derwent Publications Ltd., London, GB; AN 88-336299 & SU,A,1 394 142 (BELO MOTHER CHILD) 7 May 1988
- FERTILITY AND STERILITY, vol.36, no.1, July 1981, BIRMINGHAM US pages 61 - 67 C.H.WU ET AL.

## Description

This invention relates to methods and devices for the assaying of analytes in urine.

For the purpose of providing awareness of the status of the mammalian, eg, human, ovulation cycle, either to promote or to reduce the likelihood of conception, the measurement of the concentration of estradiol or a metabolite thereof (usually, in humans, estrone-3-glucuronide - "E3G") is often recommended, as it is well known that the body fluid concentrations of members of this analyte group rise several days in advance of ovulation, and are associated with changes in the female reproductive tract which enable the gametes to meet. To be useful, such concentration data must be determined accurately, usually from a series of samples. For example, a sample may need to be collected daily over an extended sequence of days, and successive daily analyte concentrations compared to identify a significant concentration change indicative of a change in fertility (ie. fecundity) status.

A natural variable that can interfere with the comparability of such concentration data is the "volume", or relative concentration, of the original body fluid source from which the assay sample is taken. This is particularly important and significant in relation to urine, which is most commonly chosen as the body fluid appropriate for such assays. The necessary sample is collected while urine is being excreted. However, when the collected sample is analyzed for the presence of a specific analyte, such as E3G, the apparent concentration of the analyte may not be a true reflection of the amount of analyte being produced by the body at that time. The degree of fluid intake, and kidney function, has a very significant influence on the actual volume, and frequency, of urine excretion. If fluid intake has been relatively high, or relatively low, during the previous few hours, the measurable concentration of analyte in the collected sample can be much lower (or higher) than normal, leading to inaccurate and possibly misleading information.

For the purposes of this specification, we shall refer to such natural variation in body fluid source concentration as "biological volume variability".

There is a clear need for a method of obtaining such concentration data in a manner which is less influenced by biological volume variability. To achieve this objective, it is desirable to identify another component of the body fluid source which can be analysed readily and which can be correlated somehow with the biological volume of the source. Previously, the concentration of creatinine has been proposed as a "volume corrector", in laboratory assays. Creatinine has the disadvantage that it is excreted in human urine in relatively large amounts, compared to E3G, and its analysis would normally require dilution of the collected urine sample, and usually require the use of a different type of assay. Dilution of the sample would conflict with the necessary accuracy of the E3G assay, and it is difficult to configure a comprehensive assay for the volume corrector and the E3G in a single collected sample.

We have discovered that androgens and their metabolites, most particularly testosterone and testosterone-17β-glucuronide ("T17G"), can be used very effectively as "volume correctors". They can be used in this role in the quantitative assaying of analytes, especially steroid analytes, in urine. They are particularly useful in this role in the quantitative assaying of body fluid concentrations of estradiol and its metabolites. In particular, we have noted that the profile of the ratio between the urinary concentrations of E3G and T17G exhibits a peak similar to the peak in the urinary E3G profile during the pre-fertile phase of the human ovulation cycle, and that by observing the rise or peak in the value of this ratio during this phase, a useful warning of imminent ovulation (and hence the time of fecundity or potential fertility) can be given despite the biological volume variability that may be occurring in the source of body fluid sample.

T17G is excreted in human urine in amounts similar to those associated with estradiol metabolites such as E3G, and originates from similar metabolic processes from the parent hormone. Furthermore, the concentration of T17G in urine can easily be measured even when the collected urine sample is undiluted, thus facilitating a combined assay for these two components.

WPI abstract AN 88-336299 (SU 1394142) describes the measurement of estreiol in infant blood plasma, and uses the supplementary determination of testosterone to increase accuracy. EP-A-086095 describes assays for determining the ratio of estrone-e-glucuronide and pregnanediol-3-glucuronide in urine. Neither document suggests that an androgen such as testosterone might be useful as a volume corrector in urine assays.

By the invention we provide a method for determining in absolute or relative terms the concentration of an analyte, especially estradiol or a metabolite thereof in a urine sample, wherein the method is rendered less dependent on biological volume variability by determining the absolute or relative concentration of an androgen or a metabolite thereof in the same original urine sample source, preferably the same collected sample of urine, and comparing the two concentration values so determined.

For the purposes of illustration only, the invention will be described as applied in the assaying of estradiol metabolites such as E3G.

For the objectives of the invention to be achieved it is essential that the assay for the analyte and the volume corrector must be conducted on body fluid that has been sampled from the same original source, i.e. from the same event of urine excretion. Two or more samples may be collected from the original source, and assayed separately if desired, but it is more convenient, and in general more accurate, if a single sample is taken and assayed for both of the analyte and volume corrector (if necessary or conveniently, following subdivision of the single collected sample).

Preferably, the androgen metabolite is testosterone-17β-glucuronide. Other particularly useful androgen metabolites aare epitestosterone glucuronide (T17αG) and 5α-dihydrotestosterone (5αT17βG).

The invention is particularly applicable where the concentration of estrone-3-glucuronide, as an estradiol metabolite, is measured.

In addition to estrone-3-glucuronide, estradiol metabolites that can also be assayed for the purposes of the invention include estradiol-3-glucuronide, estradiol-17β-glucuronide, estriol-3-glucuronide, estriol-16α-glucuronide and (principally for non-human subjects) estrone-3-sulphate and estradiol-17β-sulphate. As will be appreciated from the following description, the invention can readily be applied to data derived from the measurement of urinary concentrations of other analytes of significance in relation to the status of the ovulation cycle, where these exhibit a comparable relationship with the concentration of a measurable androgen or metabolite thereof that has undergone similaar metabolic processes. Generally, the most suitable analytes are hormones and their metabolites.

The skilled reader will also appreciate that the urinary "concentration" of the chosen analyte or analytes, and volume corrector, need not be measured in absolute terms, although this can of course be done if desired. Generally, it will be sufficient to assay an analyte in a manner which yields a signal, convertible to numerical data, related to the actual concentration, so that such data can be compared with similar data obtained at a different stage in the cycle to determine whether or not a significant change in actual concentration has occurred. Accordingly, where the specification and claims below refer to the "concentration" of an analyte, this expression should be interpreted broadly.

Conveniently, the analytical result can be expressed as a ratio of the two concentrations or signals.

An important embodiment of the invention is a method of monitoring the concentration of estradiol or a metabolite thereof in the urine of a human female subject, involving successive (e.g. daily over a plurality of days during a current cycle) sampling of the urine of said subject and measuring the estradiol/metabolite concentration in each urine sample, wherein the concentration data so obtained is rendered less dependent on biological volume variability by determining the concentration of an androgen or a metabollite thereof in each original urine source, preferably the same collected urine sample, as a reference against which to compare the measured estradiol/metabolite concentration. Preferably, the estradiol metabolite measured is E3G. Preferably, the androgen metabolite measured is T17G. The parent hormones estradiol and testosterone are preferred for the analysis of saliva andd vaginal fluid.

Another embodiment of the invention is a method of providing awareness of the status of the ovulation cycle of an individual mammalian, e.g. human, female subject, involving the detection of the rise in urinary concentration of estradiol or a metabolite thereof (eg E3G) indicative of imminent ovulation, wherein the urinary concentration of an androgen or a metabolite thereof, especially T17G, in the same subject is measured and used to render the estradiol (metabolite) concentration data less dependent on biological volume variability. Preferably, in a human subject, awareness of the status of the ovulation cycle is provided by detecting a rise and/or peak in the E3G/T17G ratio indicative of imminent ovulation.

By observing a rise in E3G/T17G ratio, early prediction of ovulation can be provided. The peak E3G/T17G ratio occurs at about the same time in the cycle as the peak concentration of luteinising hormone (LH) in the body fluid. The time for fecundity (potential fertility) can be determined from a defined rise in the ratio and a calculated interval from the peak.

The invention also provides an assay device for determining the concentration of estradiol or a metabolite thereof, especially E3G, in a sample, which device also enables the concentration of an androgen or a metabolite thereof, especially T17G, in the same urine sample to be determined.

An additional advantage of using the measurement of the urinary concentration of E3G and T17G in combination as the basis of a fertility awareness test procedure is that the need to pin-point the event of actual ovulation by the measurement of a further analyte, such as luteinizing hormone (LH) or a pregnanediol-3-glucuronide (P3G) is avoided The E3G/T17G ratio peak can be used as a sufficient indicator alone. Ovulation will occur within an interval of 12 to 48 hours, generally about 24 hours, following occurrence of the E3G/T17G ratio peak.

Methods of detecting body fluid analytes, such as urinary hormone metabolites, suitable for the purposes of this invention, are well known to those skilled in the art. In a preferred embodiment, the analyte is detected by assay methods and devices as described in our GB-A-2204398 and EP-A-383619.

Because the method of the invention relies on measurement of a urine component, this must be done on a urine sample. A variety of immunoassay techniques are available which enable urine components to be measured. A wide variety of solid phase testing devices such as dipsticks and chromatographic strips have been described in the literature, and can readily be adapted for use in determining urinary analytes. Examples of simple assay technology that can readily be adapted for use in the home is described, for example, in EP-A-0225054, EP-A-0183442, EP-A-0186799 and GB-A-2204398. Disposable assay strips such as those described in GB-A-2204398 and EP-A-383619 which simply required to contacted with urine and which provide an assay result in semi-quantitative form, e.g. by means of a series of test zones on the strip which are progressively positive at higher urinary analyte levels, can be used. Multiple strips that respond at different analyte thresholds can be used, rather than a single strip. Alternatively, a visually readable quantitative assay can be based on progression of a visible, e.g. coloured, region or "front" over a surface (e.g. radial diffusion), using for example an enzyme-labelled assay. A measure of analyte concentration can be derived by comparing a visible result, e.g. colour intensity, with a guide such as a chart or scale provided as part of an assay kit.

In a more sophisticated version of an apparatus according to the invention, the recording device can incorporate means for reading the result of the urine assay, e.g. by measuring the reflectance or fluorescence from an assay strip. This may enable a more precise numerical indication to be given of the analyte level, and further enhance the accuracy of the method.

As the invention requires that at least two analytes (the analyte of interest and the volume corrector) are measured essentially simultaneously, such measurement can if desired be performed using a single testing device, e.g. a device incorporating multiple assay strips, or a single strip capable of independently detecting the level of the different analytes.

It is generally envisaged that the determination of the concentration of the analytes relevant to this invention will be made using immunoassays. This requires the availability of highly specific (e.g. monoclonal) antibodies for the reagents. The preparation of antibodies against steroid analytes is now conventional and the familiar monoclonal antibody system which has been known since the published work of Kohler and Milstein (1975) can be used. This technology is now commonplace. By way of example only, a method by which monoclonal antibodies against E3G and T17G can be prepared is set out below. Because these steroid molecules are too small to be immunogenic in their own right, the preparation of specific antibodies against them requires the preparation of conjugates using, for example, a protein such as bovine serum albumen (BSA). The preparation of hapten-BSA conjugates is also routine technology.

### Example 1

By way of example only, some practical aspects of the invention are described below with reference to the accompanying drawings, of which:
Figure 1 of the accompanying drawings illustrates an ovulation cycle monitoring device for use in accordance with the invention, together with an associated urine sample testing device.
Figure 2 shows the urine testing device in greater detail. Figure 3 shows, in schematic form, the basic functions that may be required in an electronic monitor for use in accordance with the invention.

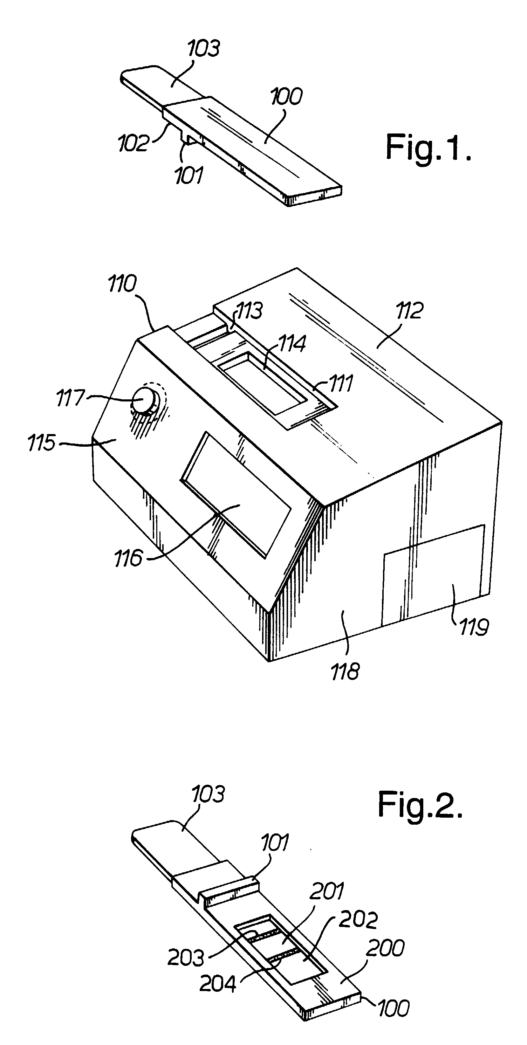

Referring to Figure 1, the urine sample testing device comprises a flat elongate casing 100 having a locating means, represented by ridge 101 on its lower surface 102. Projecting from one end of the casing is a bibulous sample receiving member 103.

The monitor comprises a casing 110 having a recess 111 in its upper surface 112 to accommodate the casing 100 of the testing device. Recess 111 incorporates a locating slot 113 into which the locating ridge 101 on the casing of the testing device can be inserted to positively locate the testing device in relation to a reading window 114 in the recess. Casing 110 contains means (not shown) such as a fluorescent reader or optical density reader to measure the result of a simultaneous urinary analyte concentration assay and volume corrector concentration assay performed using the testing device, according to the labelling system chosen to reveal the assay result.

The sloping front face 115 of the monitor casing incorporates a large window 116 through which information can be conveyed to the user e.g. by means of an LED display or other visual output. This information can be provided in a variety of forms, such as an indication of a calender and the need to perform urine tests, and an indication of the current status of the ovulation cycle. The sloping face 115 of the casing also incorporates a button 117 which the user can press to indicate the commencement of an ovulation cycle and to start the monitor processing information relative to that cycle.

Information can be conveyed to the user by means of a liquid crystal or LED display, for example. If desired, information on the state of fertility can be conveyed by a simple visual indication, e.g. a combination of colours showing for example, green for infertile and red for fertile. Optionally, another signal, e.g. yellow, for any intermediate stage when conception is less likely but still possible, may be shown. Especially if the device is intended primarily as an aid to contraception, it should "fail safe" by showing a "fertile" signal.

In general the monitor will be battery-powered, and incorporates in side 118 of the casing an access point such as a removable cover 119 to permit batteries to be inserted and changed.

Referring to Figure 2, the testing device is shown inverted relative to the aspect seen in Figure 1. The locating ridge 101 is now on the upper surface 200. Also in the surface 200 now uppermost is a result window 201. The body of the testing device can incorporate an immunochromatographic strip 202 partially visible through the result window, incorporating all necessary reagents to enable immunoassays to be performed which detect the presence and concentration of analyte in a urine sample appied to the sample collecting member 103, and also volume corrector in the same applied sample. The results of the assays can be effected by the immobilization of a labelled component, via sandwich or more preferably competition reactions in the presence of analyte and volume corrector in an applied urine sample, the labelled reagents becoming concentrated in two zones 203 and 204 revealed through the result window. When the testing device is inverted and located in the recess 111 in the casing of the monitor, the result window is immediately adjacent to the reading window 114 in the monitor and the assay results can be determined. For example, if the label is a fluorescent reagent, the reading means within the monitor can detect and measure fluorescent light output from accumulated label in the detection zones 203 and 204 on the strip to provide numerically accurate concentration values for the analyte and volume corrector in the urine sample. This information can be processed by the monitor together with calender information resulting from the initiation of the cycle process by the user and historical data which the monitor can retain from previous cycles.

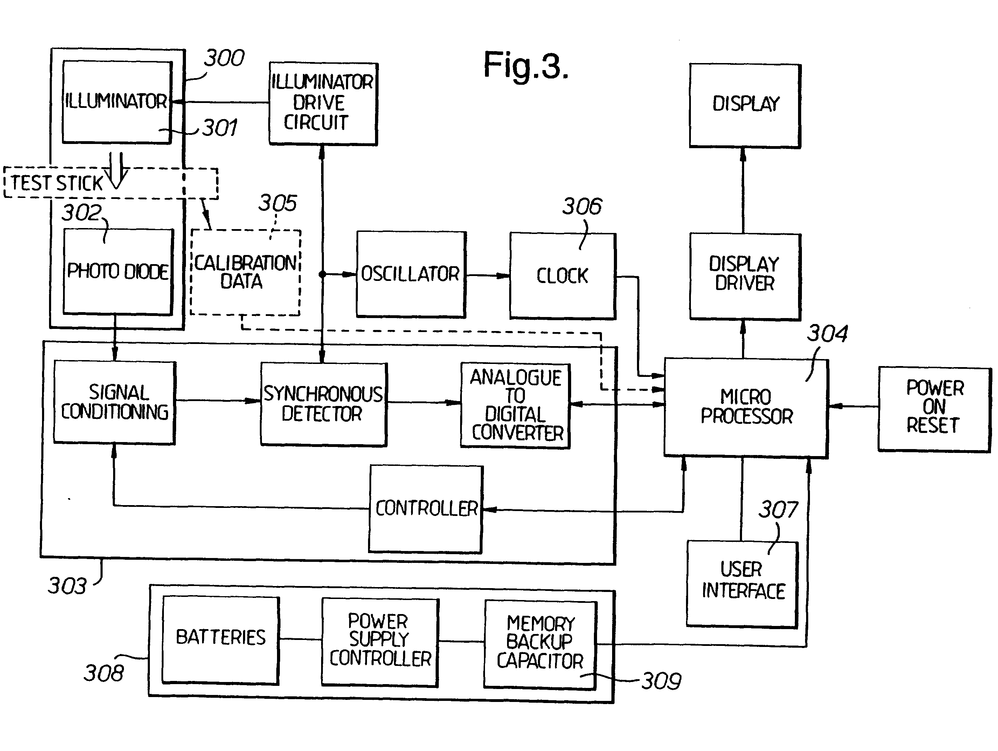

Referring to Figure 3, some of the basic elements which may be required in an electronic monitoring device are seen. The individual features can be entirely conventional, and those familiar with the art of electronics will appreciate that other combinations and arrangements of such features can be employed to achieve the objectives of the invention. For example, so-called "hard-wired" systems, and "neural networks", can be used in place of conventional microprocessors based on "chip" technology. As depicted in Figure 3, the combination essentially comprises:

A reading unit 300 to derive information from a test device, such as a test stick, the reading unit comprising an illuminator 301 and a reader 302 (represented here as a photo diode). The reading unit feeds into a conversion unit 303 to convert the optical signal into a form usable by a microprocessor 304. As an optional feature, a calibration system 305 is provided to convert signals derived from the reading unit into data corresponding, for example, to absolute concentration values. A timer, such as a clock 306 is required to regulate measurements within a cycle. The microprocessor 304 processes, memorizes and interprets results in the light of previous events, particularly recorded from previous cycles. The user interface 307 will generally comprise at least means, such as a push button, which the user can operate at the commencement of a cycle to initiate the operation of the device as a whole. The power supply 308 should include means, such as a memory back up capacitator 309, to prevent loss of historical data if it becomes necessary to replace batteries.

A urine sampling and testing device, having an external appearance as shown in Figure 2 and functioning as described above, can be made from the following basic components:
a hollow casing;
an immunochromatographic strip, e.g. of nitrocellulose, having two test zones comprising respectively a line or other defined region of immobilised anti-E3G monoclonal antibody and a separate line or region of immobilised anti-T17G monoclonal antibody;
a bibulous urine sample collector which can feed collected sample into the strip; and
labelled E3G and labelled T17G reagents, contained within the casing, e.g. in the dry state, such that applied urine sample can take up both reagents and competition reactions between these reagents and E3G and T17G in the urine can occur and the extent to which the labelled reagents become bound in their respective test zones being inversely proportional to the amount of E3G and T17G in the urine sample.

The label (which can be the same or different on the two reagents) can for example be a fluorescent compound, a dye sol, a metallic (e.g. gold) sol, a non-metallic elemental sol (e.g. selenium), or a coloured insoluble particle such as coloured latex.

If desired, the device can incorporate a "control" zone or other indication to tell the user the assay has been completed satisfactorily and the device can be presented to the monitor for reading and interpretation.

If desired, the device can also contain standard reagents or other means enabling the monitor to calibrate the assay results.

These devices will usually be disposable after individual use. Conveniently they can be supplied to the user in replenishment packs containing a substantial quantity of the devices, with instructions for use in conjunction with the monitor that the user has retained from previous cycles.

The invention includes an electronic device or monitor programmed for use in a method as generally described above, and also a test kit comprising such a programmed device or monitor together with one or more assay devices as generally described above.

### Example 2

This example uses laboratory techniques to demonstrate the principle of the invention.

### Preparation of T17G-BSA conjugate

To steroid (20mg) dissolved in 500µl dimethyl formamide were added two molar equivalents of tri-n-butylamine. The solution was allowed to stand for 10 minutes at 10°C, after which time one molar equivalent of isobutyl chloroformate was added. The solution was stirred for 35 minutes at 10°C before adding to a pre-cooled solution of BSA in water (25mg in 1.8 ml) which had been adjusted to pH 9.5, with 1 M sodium hydroxide; upon addition of the reaction mixture the pH was maintained at approximately 8.0 by addition of 1 M sodium hydroxide. The solution was stirred at 4°C for 4 hours. Unreacted steroid was removed by dialysis against several changes of 0.1 M phosphate buffer, pH 7.4, containing Norit A charcoal.

The identical procedure was used to prepare an E3G-BSA conjugate.

### Monoclonal antibody production:

Mouse monoclonal anti-Testosterone-17-glucuronide antibodies wre prepared by repeated immunization of BALB/c mice with T17G-BSA conjugates, followed by fusion of their spleen cells with a non-secreting mouse myeloma line NSO. Alternatively, the commercially-available hybridoma in SP2/0 can be used. Hybridomas secreting anti-T17G antibodies were detected by enzyme immunoassay. Hybridomas were cloned by limiting dilution and grown ascites. Growth in serum-free medium in vitro is an alternative. The antibodies were purified by chromatography on protein A-sepharose.

### Conjugation of steroid glucuronides to alkaline phosphatase:

To steroid glucuronide dissolved in dimethyl formamide (1mg/ml) were added two molar equivalents of tri-n-butylamine (5µl/ml in dimethyl formamide) and the mixture was cooled to 10°C for 10 minutes. Then 1 molar equivalent of isobutyl chloroformate (5µl/ml in dimethyl formamide) was added. This was left for 35 minutes at 10°C, after which time 350µl of the reaction mixture were transferred to a solution of alkaline phosphatase (0.5mg) in 1ml 0.5% sodium bicarbonate. The mixture was left for 2 hours at 4°C. Unreacted steroid was removed by diaylysis of the reaction mixture against 1 litre 0.1M phosphate buffer, pH7.4, containing 4g Norit A charcoal, for 48 hours. After dialysis, the conjugate was added to 5ml 0.05 M Tris buffer, pH 8.0, containing ovalbumin (50mg/ml), 0.01 M magnesium chloride and 0.05% sodium azide.

### Measurement of T17G in Urine:

Aliquots (200µ) of anti-testosterone 17-glucuronide (T17G) antibody diluted in pH 7.2 phosphate-buffered saline, containing 0.15% Tween™20 and 0.01% sodium azide (PBSTA) were incubated for 1 hour at room temperature with nylon pegs sensitised with rabbit anti-mouse IgG. After washing in PBSTA, the pegs were placed in microtitre wells containing 100µl T17G-alkaline phosphatase conjugate and 100µl of urine sample, and incubated for 1 hour. After rewashing, the pegs were incubated with 200µl - p-nitrophenol substrate solution for 1 hour and the optical densities read at 405nm after removing the pegs. Calibration graphs were constructed with pure T17G standards in place of the urine samples.

### Measurement of E3G in urine

Exactly the same method as above, replacing the T17G antibody and other reagents with E3G reagents.

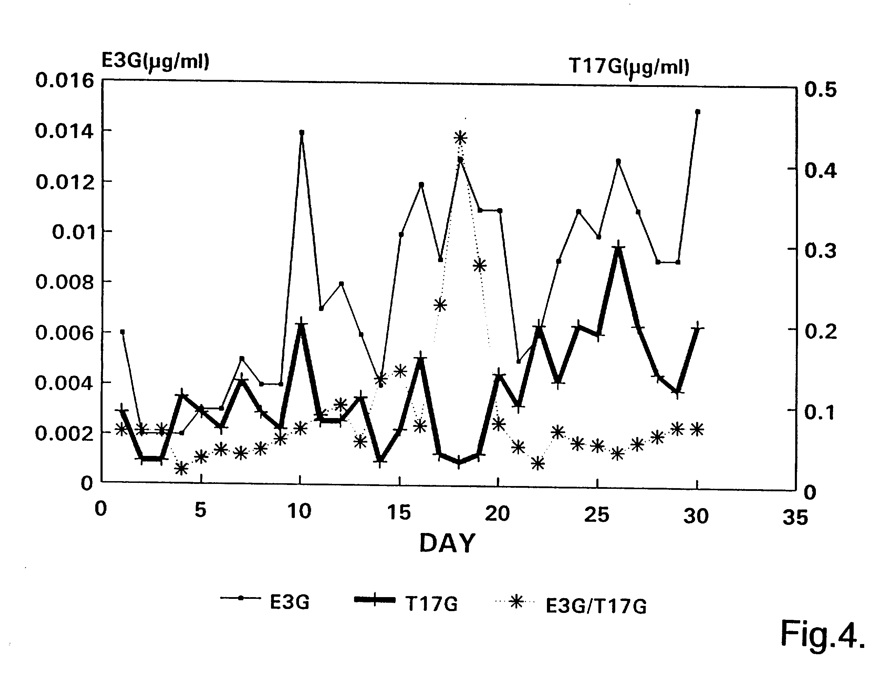

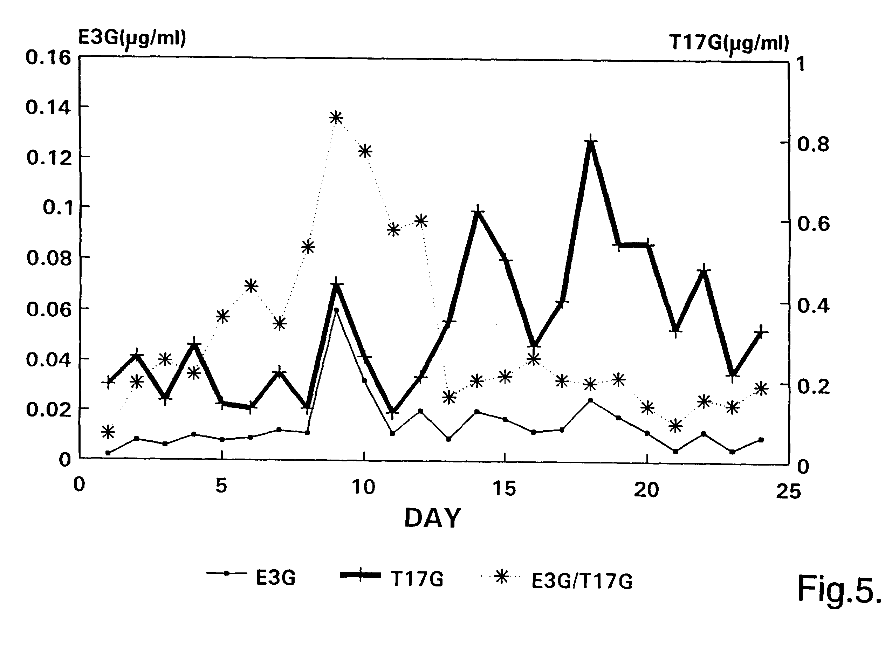

Figures 4 and 5 of the accompanying drawings show profiles of E3G, T17G and the ratio of the two analytes during two ovulation cycles in one individual, from measurements made as set forth above. In each cycle, despite considerable fluctuation in the concentrations of the individual analytes, attributed largely to biological volume variability, there is a clear single peak in the ratio value.

## Claims

1. A method for determining in absolute or relative terms the concentration of an analyte in a urine sample, wherein the method is rendered less dependent on biological volume variability in the original sample source by determining the absolute or relative concentration of an androgen or a metabolite thereof in the same original urine sample source and comparing the two concentration values so determined.

2. A method according to claim 1, wherein the analyte is estradiol or a metabolite thereof, such as estrone-3-glucuronide.

3. A method according to claim 1 or claim 2, wherein the concentration of testosterone or testosterone-17β-glucuronide is measured.

4. A method according to any one of the preceding claims, wherein the result is expressed as a ratio of the two concentrations.

5. A method of monitoring the concentration of estradiol or a metabolite thereof, such as E3G, in the urine of a human female subject, involving successive sampling of the urine of said subject and measuring the estradiol/metabolite concentration in absolute or relative terms in each urine sample, wherein the concentration data so obtained is rendered less dependent on biological volume variability in the original urine source by determining the absolute or relative concentration of an androgen or a metabolite thereof, such as T17G, in the each same original urine source as a reference against which to compare the measured estradiol/metabolite concentration.

6. A method of providing awareness of the status of the ovulation cycle of an individual human female subject, involving the detection of the rise in the urinary E3G concentration indicative of imminent ovulation, wherein the urinary concentration of an androgen or a metabolite thereof, especially T17G, in the same subject is measured in absolute or relative terms and used to render the E3G concentration data less dependent on urine biological volume variability.

7. A method according to claim 6, wherein awareness of the status of the ovulation cycle is provided by detecting a rise in the E3G/T17G ratio indicative of imminent ovulation.

8. An assay device which provides a readable signal indicative of the concentration of estradiol or a metabolite thereof, especially E3G, in a urine sample, which device also provides a readable signal indicative of the concentration of an androgen or a metabolite thereof, especially T17G, in the same urine sample.

9. A test kit comprising one or more assay devices according to claim 8, together with an electronic device or monitor incorporating means for reading one of said devices and deriving therefrom an indication of said concentration of estradiol or said metabolite thereof.

10. An electronic device or monitor programmed to derive an indication of the urinary concentration of an analyte from essentially simultaneous measurements in absolute or relative terms of the concentrations of said analyte and of an androgen or a metabolite thereof, especially T17G, in a single urine sample.

## Patentansprüche

1. Verfahren zur Bestimmung der Konzentration eines Analyten in einer Urinprobe in absoluter oder relativer Hinsicht, worin das Verfahren von der biologischen Volumenvariabilität in der ursprünglichen Probenquelle weniger abhängig gemacht wird, indem die absolute oder relative Konzentration eines Androgens oder eines seiner Metaboliten in derselben ursprünglichen Urinprobenquelle bestimmt und die beiden so bestimmten Konzentrationswerte verglichen werden.

2. Verfahren nach Anspruch 1, worin der Analyt Estradiol oder einer seiner Metaboliten, wie Estron-3-glucuronid, ist.

3. Verfahren nach Anspruch 1 oder 2, worin die Konzentration von Testosteron oder Testosteron-17ß-glucuronid gemessen wird.

4. Verfahren nach irgendeinem der vorhergehenden Ansprüche, worin das Ergebnis als ein Verhältnis der beiden Konzentrationen ausgedrückt wird.

5. Verfahren zur Überwachung der Konzentration von Estradiol oder einem seiner Metaboliten, wie E3G, im Urin eines menschlichen weiblichen Individuums, umfassend die aufeinanderfolgende Probennahme des Urins des Individuums und Messen der Estradiol/Metabolit-Konzentration in absoluter oder relativer Hinsicht in jeder Urinprobe, wobei die so erhaltenen Konzentrationswerte in der ursprünglichen Urinquelle von der biologischen Volumenvariabilität weniger abhängig gemacht werden, indem die absolute oder relative Konzentration eines Androgens oder eines seiner Metaboliten, wie T17G, jeweils in derselben ursprünglichen Urinquelle als Referenz bestimmt wird, mit der die gemessene Estradiol/Metabolitkonzentration zu vergleichen ist.

6. Verfahren, um Kenntnis über den Zustand des Ovulationszyklus eines einzelnen menschlichen weiblichen Individuums zu erlangen, umfassend den Nachweis der Zunahme der die bevorstehende Ovulation anzeigenden Urin-E3G-Konzentration, wobei die Urinkonzentration eines Androgens oder eines seiner Metaboliten, insbesondere T17G, im selben Individuum in absoluter oder relativer Hinsicht gemessen und verwendet wird, um die E3G-Konzentrationswerte von der biologischen Volumenvariabilität im Urin weniger abhängig zu machen.

7. Verfahren nach Anspruch 6, worin die Kenntnis des Zustands des Ovulationszyklus durch Nachweis einer Zunahme im die bevorstehende Ovulation anzeigenden E3G/T17G-Verhältnis erlangt wird.

8. Nachweisvorrichtung, die ein die Konzentration von Estradiol oder einem seiner Metaboliten, insbesondere E3G, in einer Urinprobe anzeigendes ablesbares Signal liefert, wobei die Vorrichtung ebenfalls ein die Konzentration eines Androgens oder eines Metaboliten hiervon, insbesondere T17G, in derselben Urinprobe anzeigendes ablesbares Signal liefert.

9. Testkit, umfassend ein oder mehrere Nachweisvorrichtungen nach Anspruch 8, zusammen mit einer elektronischen Vorrichtung oder einem Überwachungsgerät, das Mittel zum Ablesen für eine der Vorrichtungen und daraus Ableiten einer Anzeige der Konzentration von Estradiol oder einem seiner Metaboliten aufweist.

10. Elektronische Vorrichtung oder Überwachungsgerät, das programmiert ist, um eine Anzeige der Urinkonzentration eines Analyten aus im wesentlichen gleichzeitigen Messungen in absoluter oder relativer Hinsicht der Konzentrationen des Analyten und eines Androgens oder eines seiner Metaboliten, insbesondere T17G, in einer einzelnen Urinprobe abzuleiten.

## Revendications

1. Procédé de détermination en termes absolus ou relatifs de la concentration d'un analyte dans un échantillon d'urine, dans lequel le procédé est rendu moins dépendant de la variabilité du volume biologique dans la source d'origine de l'échantillon en déterminant la concentration absolue ou relative d'un androgène ou de l'un de ses métabolites dans la même source d'origine de l'échantillon d'urine et en comparant les deux valeurs de concentration ainsi déterminées.

2. Procédé selon la revendication 1, dans lequel l'analyte est l'oestradiol ou l'un de ses métabolites tel que l'oestrone-3-glucuronide.

3. Procédé selon la revendication 1 ou la revendication 2, dans lequel on mesure la concentration en testostérone ou en testostérone-17β-glucuronide.

4. Procédé selon l'une quelconque des revendications précédentes, dans lequel le résultat est exprimé comme un rapport des deux concentrations.

5. Procédé de monitorage de la concentration d'oestradiol ou de l'un de ses métabolites tel que l'E3G dans l'urine d'un sujet humain de sexe féminin, comprenant les étapes consistant à effectuer des prélèvements successifs de l'urine dudit sujet et à mesurer la concentration en oestradiol/métabolite en termes absolus ou relatifs dans chaque échantillon d'urine, dans lequel les données de concentration ainsi obtenues sont rendues moins dépendantes de la variabilité du volume biologique de la source d'origine de l'urine en déterminant la concentration absolue ou relative d'un androgène ou de l'un de ses métabolites tel que le T17G dans chaque source d'origine de l'urine pour servir de référence par rapport à laquelle on compare la concentration d'oestradiol/métabolite mesurée.

6. Procédé permettant de connaître l'état du cycle d'ovulation d'un sujet humain de sexe féminin individuel, comprenant la détection de l'accroissement de la concentration urinaire d'E3G indiquant l'imminence de l'ovulation, dans lequel on mesure en termes absolus ou relatifs la concentration urinaire d'un androgène ou de l'un de ses métabolites, notamment le T17G, chez le même sujet et on l'utilise pour rendre les données de concentration moins dépendantes de la variabilité du volume biologique de l'urine.

7. Procédé selon la revendication 6, dans lequel la connaissance de l'état du cycle d'ovulation est donnée par la détection d'un accroissement du rapport E3G/T17G indiquant l'imminence d'une ovulation.

8. Dispositif de dosage fournissant un signal pouvant être lu et indiquant la concentration d'oestradiol ou de l'un de ses métabolites, notamment l'E3G, dans un échantillon d'urine, lequel dispositif fournit également un signal pouvant être lu et indiquant la concentration d'un androgène ou de l'un de ses métabolites, notamment le T17G, dans le même échantillon d'urine.

9. Trousse de test comprenant un ou plusieurs dispositifs de dosage selon la revendication 8 ainsi qu'un dispositif électronique ou moniteur comprenant des moyens pour lire l'un desdits dispositifs et pour en obtenir une indication de ladite concentration d'oestradiol ou de l'un de ses métabolites.

10. Dispositif électronique ou moniteur programmer pour obtenir une indication de la concentration urinaire d'un analyte à partir de mesures essentiellement simultanées en termes absolus ou relatifs des concentrations dudit analyte et d'un androgène ou de l'un de ses métabolites, notamment le T17G, dans un seul échantillon d'urine.
